# EUROPÄISCHE PATENTSCHRIFT

(11) **EP 0 556 680 B1**
(45) Veröffentlichungstag und Bekanntmachung des Hinweises auf die Patenterteilung: **17.07.1996**
(21) Anmeldenummer: 93101917.8
(22) Anmeldetag: 08.02.1993
(51) Int. Cl.: C07D 333/54, C07C 323/22

(54) **Verfahren zur Herstellung von Benzo[b]thiophenen**
Process for the preparation of benzo(b)thiophenes
Procédé de préparation de benzo(b)thiophènes

(30) Priorität: 19.02.1992 DE 4204969
(43) Veröffentlichungstag der Anmeldung: 25.08.1993
(73) Patentinhaber: BASF Aktiengesellschaft, D-67063 Ludwigshafen (DE)
(72) Erfinder: Ebel, Klaus, Dr., W-6704 Mutterstadt (DE); Schroeder, Juergen, Dr., W-6806 Viernheim (DE)

(56) Entgegenhaltungen:
- A.R.KATRITZKY AND A.J.BOULTON 'Advances in heterocyclic chemistry, Volume 11' 1970 , ACADEMIC PRESS , NEW YORK AND LONDON

## Beschreibung

Die Erfindung betrifft ein neues Verfahren zur Herstellung von Benzo[b]thiophenen durch Umsetzung von Thiophenolen mit Chloracetaldehyd zu den (Arylthio)acetaldehyden und die anschließende Cyclisierung.

Aus Adv.Heterocycl.Chem. 11, 206 bis 239 (1970) sind verschiedene Verfahren zur Herstellung von Benzo[b]thiophenen bekannt. Für eine technische Synthese sind diese Verfahren wegen ihrer teuren Einsatzstoffe bzw. ihrer schlechten Ausbeuten ungeeignet.

Aus dem Proc.Indian Acad.Sci. 32A, 390 bis 395 (1950) eine zweistufige Benzo[b]thiophensynthese bekannt. In der ersten Stufe werden Thiophenole in Gegenwart equimolarer Mengen Natriumalkoholat mit Bromacetaldehydacetalen zu (Arylthio) acetaldehydacetalen umgesetzt. Die (Arylthio) acetaldehydacetale werden dann in Polyphosphorsäure bei Temperaturen von 160 bis 180°C cyclisiert. Die Benzo[b]thiophene können kontinuierlich abdestilliert werden. Für eine technische Synthese sind die Einsatzstoffe zu teuer; außerdem fallen äquimolare Mengen Salz an.

Aus diesen Gründen hat die synthetische Herstellung von Benzo[b]thiophen bislang gegenüber der Gewinnung aus Steinkohlenteer keine Bedeutung (Ullmanns Enzyklopädie der technischen Chemie, 4.Auflage, Band 23, Seite 217 bis 225).

Der vorliegenden Erfindung lag daher die Aufgabe zugrunde, den zuvor genannten Nachteilen abzuhelfen.

Demgemäß wurde ein neues und verbessertes Verfahren zur Herstellung von Benzo[b]thiophenen der allgemeinen Formel I 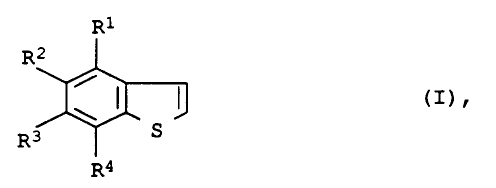
in der
- R¹,R²,R³,R⁴: unabhängig voneinander Wasserstoff, C₁- bis C₄-Alkyl, C₁- bis C₄-Alkoxy, C₁- bis C₄-Halogen-alkyl, Nitro, Cyano, Halogen, C₁- bis C₄-Alkyl-carbonyl, Benzoyl, C₁- bis C₄-Alkylcarbonylamino, Benzoylamino, N-C₁- bis C₄-Alkylphenylamino, C₁- bis C₄-Alkoxycarbonyl, C₁- bis C₄-Alkylsulfonyl, Phenylsulfonyl, Aminosulfonyl, Aminocarbonyl, C₁- bis C₄-Phenylalkyl und Nitrobenzyl oder R¹, R² oder R², R³ oder R³, R⁴ eine gegebenenfalls durch R¹ bis R⁴ substituierte Butadiendiylkette
bedeuten, gefunden, welches dadurch gekennzeichnet ist, daß man
a) Thiophenole der allgemeinen Formel II 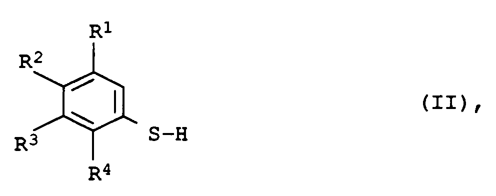 in der die Substituenten R¹ bis R⁴ die obengenannten Bedeutungen haben, mit Chloracetaldehyd bei Temperaturen von 0 bis 150°C umsetzt und
b) die entstandenen (Arylthio) acetaldehyd der allgemeinen Formel III 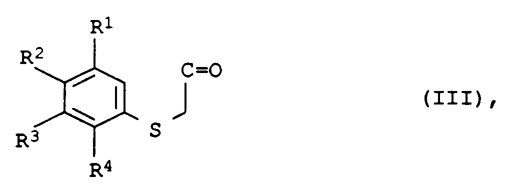 in der die Substituenten R¹ bis R⁴ die obengenannten Bedeutungen haben, durch Einleiten in Polyphosphorsäure oder ein Gemisch aus Phosphorsäure und Phosphorpentoxid bei Temperaturen von 100 bis 300°C und Drücken von 0,001 bis 1 bar cyclisiert.

Das erfindungsgemäße Verfahren läßt sich wie folgt durchführen:
a) In der ersten Stufe können die Thiophenole II mit Chloracetaldehyd, bevorzugt wäßrigem Chloracetaldehyd bei Temperaturen von 0 bis 150°C, vorzugsweise bei 40 bis 100°C umgesetzt und bevorzugt Wasser und Chlorwasserstoff mit einem geeigneten Lösungsmittel wie C₄- bis C₃₀-Alkanen oder C₄- bis C₃₀-Cycloalkanen z.B. Cyclohexan oder halogenierten Kohlenwasserstoffen z.B. Chloroform azeotrop abdestilliert.
   Die Thiophenole II und Chloracetaldehyd können im Molverhältnis von 0,1 : 1 bis 5 : 1, bevorzugt 0,5 : 1 bis 1,5 : 1, besonders bevorzugt 0,8 : 1 bis 1,2 : 1 eingesetzt werden.
b) Das in der ersten Stufe entstandene rohe (Arylthio)acetaldehyd III, wird in der zweiten Stufe vorzugsweise in dem in der ersten Stufe verwendeten Lösungsmittel bei einem Druck von 0,001 bis 1 bar, bevorzugt 0,002 bis 0,5 bar, besonders bevorzugt 0,005 bis 0,2bar, in die auf Temperaturen von 100 bis 300°C, vorzugsweise 150 bis 250°C, bevorzugt 150 bis 200°C, vorgeheizte Polyphosphorsäure oder ein Gemisch aus Phosphorsäure und Phosphorpentoxid geleitet. Zweckmäßigerweise werden Druck und Temperatur so gewählt, daß das Benzo[b]thiophen I kontinuierlich abdestilliert.

Im Vergleich zu den bekannten Verfahren liefert das erfindungsgemäße Verfahren auf einfacheren und wirtschaftlicherem Wege die Benzo[b]thiophene I in guten Ausbeuten, obwohl die (Arylthio)acetaldehyde III instabil sind (J.Chem.Soc., Chem.Commun. 513 bis 514 (1984)).

Die Substituenten R¹,R²,R³,R⁴ in den Verbindungen der Formeln I, II und III haben folgende Bedeutungen:
- R¹,R²,R³,R⁴: - unabhängig voneinander
- Wasserstoff,
- C₁- bis C₄-Alkyl wie Methyl, Ethyl, n-Propyl, iso-Propyl, n-Butyl, iso-Butyl, sec.-Butyl und tert.-Butyl, bevorzugt Methyl, Ethyl, n-Propyl und iso-Propyl, besonders bevorzugt Methyl und Ethyl,
- C₁- bis C₄-Alkoxy, wie Methoxy, Ethoxy, n-Propoxy, iso-Propoxy, n-Butoxy, iso-Butoxy, sec.-Butoxy und tert.-Butoxy, bevorzugt Methoxy, Ethoxy, n-Propoxy und iso-Propoxy, besonders bevorzugt Methoxy und Ethoxy,
- C₁- bis C₄-Halogenalkyl, bevorzugt C₁- bis C₄-Fluor, Chlor- und/oder Bromalkyl wie Fluormethyl, Difluormethyl, Trifluormethyl, Chlormethyl, Dichlormethyl, Trichlormethyl, Brommethyl, Dibrommethyl und Tribrommethyl,
- Nitro,
- Cyano,
- Halogen wie Fluor, Chlor, Brom und Jod, bevorzugt Fluor, Chlor und Brom, besonders bevorzugt Chlor und Brom,
- C₁- bis C₄-Alkylcarbonyl wie Methylcarbonyl, Ethylcarbonyl, n-Propylcarbonyl, iso-Propylcarbonyl, n-Butylcarbonyl, iso-Butylcarbonyl, sec.-Butylcarbonyl und tert.-Butylcarbonyl, bevorzugt Methylcarbonyl, Ethylcarbonyl, n-Propylcarbonyl und iso-Propylcarbonyl, besonders bevorzugt Methylcarbonyl und Ethylcarbonyl,
- Benzoyl,
- C₁- bis C₄-Alkylcarbonylamino wie Methylcarbonylamino, Ethyl- carbonylamino, n-Propylcarbonylamino, iso-Propylcarbonylamino, n-Butylcarbonylamino, iso-Butylcarbonylamino, sec.-Butylcarbonyl- amino und tert.-Butylcarbonylamino, bevorzugt Methylcarbonylamino, Ethylcarbonylamino, n-Propylcarbonylamino und iso-Propylcarbonylamino, besonders bevorzugt Methylcarbonylamino und Ethylcarbonylamino,
- Benzoylamino,
- N-C₁- bis C₄-Alkylphenylamino wie Methylphenylamino, Ethylphenyl- amino, n-Propylphenylamino, iso-Propylphenylamino, n-Butylphenylamino, iso-Butylphenylamino, sec.-Butylphenylamino und tert.-Butylphenylamino, bevorzugt Methylphenylamino, Ethylphenylamino, n-Propylphenylamino und iso-Propylphenylamino, besonders bevorzugt Methylphenylamino und Ethylphenylamino,
- C₁- bis C₄-Alkoxycarbonyl wie Methoxycarbonyl, Ethoxycarbonyl, n-Propoxycarbonyl, iso-Propoxycarbonyl, n-Butoxycarbonyl, iso-Butoxycarbonyl, sec.-Butoxycarbonyl und tert.-Butoxycarbonyl, bevorzugt Methoxycarbonyl, Ethoxycarbonyl, n-Propoxycarbonyl und iso-Propoxycarbonyl, besonders bevorzugt Methoxycarbonyl und Ethoxycarbonyl,
- C₁- bis C₄-Alkylsulfonyl wie Methylsulfonyl, Ethylsulfonyl, n-Propylsulfonyl, iso-Propylsulfonyl, n-Butylsulfonyl, iso-Butylsulfonyl, sec.-Butylsulfonyl und tert.-Butylsulfonyl, bevorzugt Methylsulfonyl, Ethylsulfonyl, n-Propylsulfonyl und iso-Propylsulfonyl, besonders bevorzugt Methylsulfonyl und Ethylsulfonyl,
- Phenylsulfonyl,
- Aminosulfonyl,
- Aminocarbonyl,
- C₁- bis C₄-Phenylalkyl wie Benzyl 1-Phenethyl und 2-Phenethyl und
- Nitrobenzyl oder
- R¹, R² oder R², R³ oder R³, R⁴: - gemeinsam
- eine gegebenenfalls durch R¹ bis R⁴ substituierte Butadiendiylkette wie in 1-Thionaphthol und 2-Thionaphthol.

Benzo[b]thiophene sind wertvolle Zwischenprodukte bei der Herstellung von Pflanzenschutzmittel und Pharmazeutika (Ullmanns Enzyklopädie der technischen Chemie, 4. Auflage, Band 23, Seite 217 bis 225).

### Beispiele

### Beispiel 1

### 1. Stufe:

In einem 2 l Vierhalskolben mit Rührer, Thermometer, Tropftrichter und Wasserabscheider werden 220 g Thiophenol und 280 g Cyclohexan vorgelegt. Bei Raumtemperatur werden unter Rühren langsam 314 g einer 50%igen wäßrigen Chloracetaldehydlösung zugetropft. Anschließend wird das Wasser azeotrop abdestilliert. Die Rohlösung (ca. 45%ig) wird ohne Reinigung für die zweite Stufe eingesetzt. Die Ausbeute beträgt 80 %.

### 2. Stufe:

In einem 500 ml Vierhalskolben mit Rührer, Thermometer, Tropftrichter und Destillationsbrücke werden 100 g Polyphosphorsäure vorgelegt. Bei 160°C und 0,1 bar wird die Rohlösung aus der ersten Stufe zugetropft. Man erhält 190 g Destillat mit der Zusammensetzung 35Fl.-% Thiophenol, 60 Fl.-% Benzo[b]thiophen und 2 Fl.-% (Phenylthio)acetaldehyd. Das Cyclohexan wird in einer Kühlfalle kondensiert. Das Rohprodukt wird destilliert. Man erhält 92 g (44 %) Benzo[b]thiophen von Siedepunkt 115°C/40 mbar in einer Reinheit von 98 Fl.-%.

### Beispiel 2

### 1. Stufe:

Man verfährt wie im Beispiel 1, setzt jedoch 248 g 4-Thiokresol ein. Die Rohlösung (ca. 50%ig) wird ohne Reinigung für die zweite Stufe eingesetzt. Die Ausbeute beträgt 85%.

### 2. Stufe:

Man verfährt wie im Beispiel 1, cyclisiert aber bei 160°C/5 mbar. Man erhält 208 g Rohdestillat mit der Zusammensetzung 20 Fl.-% 4-Thiokresol, 63 Fl.-% 5-Methyl-benzo[b]thiophen und 15 Fl.-% (4-Methylphenylthio)acetaldehyd. Das Rohprodukt wird destilliert. Man erhält 133 g (53%) 5-Methylbenzo[b]thiophen von Siedepunkt 102°C/32mbar in einer Reinheit von 98 Fl.-%.

## Patentansprüche

1. Verfahren zur Herstellung von Benzo[b]thiophenen der allgemeinen Formel I 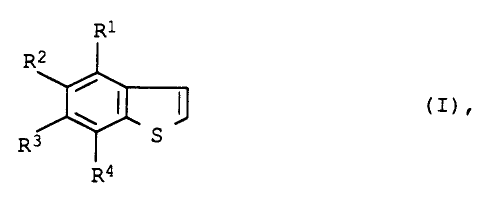 in der
R¹,R²,R³,R⁴ unabhängig voneinander Wasserstoff, C₁- bis C₄-Alkyl, C₁- bis C₄-Alkoxy, C₁- bis C₄-Halogenalkyl, Nitro, Cyano, Halogen, C₁-bis C₄-Alkylcarbonyl, Benzoyl, C₁- bis C₄-Alkylcarbonylamino, Benzoylamino, N-C₁-bis C₄-Alkylphenylamino, C₁- bis C₄-Alkoxycarbonyl, C₁- bis C₄-Alkylsulfonyl, Phenylsulfonyl, Aminosulfonyl, Aminocarbonyl, C₁-bis C₄-Phenylalkyl und Nitrobenzyl oder R¹, R² oder R², R³ oder R³, R⁴ eine gegebenenfalls durch R¹ bis R⁴ substituierte Butadiendiylkette bedeuten,
dadurch gekennzeichnet, daß man
a) Thiophenole der allgemeinen Formel II 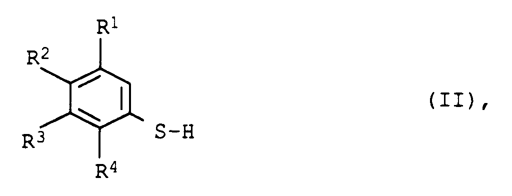 in der die Substituenten R¹ bis R⁴ die obengenannten Bedeutungen haben, mit Chloracetaldehyd bei Temperaturen von 0 bis 150°C umsetzt und
b) die entstandenen (Arylthio)acetaldehyd der allgemeinen Formel III 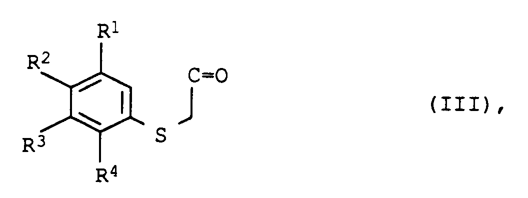 in der die Substituenten R¹ bis R⁴ die obengenannten Bedeutungen haben, durch Einleiten in Polyphosphorsäure oder ein Gemisch aus Phosphorsäure und Phosphorpentoxid bei Temperaturen von 100 bis 300°C und Drücken von 0,001 bis 1 bar cyclisiert.

2. Verfahren zur Herstellung von Benzo[b]thiophenen der allgemeinen Formel I nach Anspruch 1, dadurch gekennzeichnet, daß man aus der Reaktionsmischung Wasser azeotrop abdestilliert.

3. Verfahren zur Herstellung von Benzo[b]thiophenen der allgemeinen Formel I nach Anspruch 1, dadurch gekennzeichnet, daß man zur Cyclisierung Polyphorphorsäure verwendet.

4. Verfahren zur Herstellung von Benzo[b]thiophenen der allgemeinen Formel I nach Anspruch 1, dadurch gekennzeichnet, daß man zur Cyclisierung ein Gemisch aus Phosphorsäure und Phosphorpentoxid verwendet.

## Claims

1. A process for the preparation of benzo[b]thiophenes of the general formula I 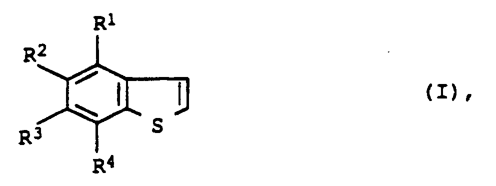 where
R¹, R², R³, R⁴ are, independently of one another, hydrogen, C₁-C₄-alkyl, C₁-C₄-alkoxy, C₁-C₄-haloalkyl, nitro, cyano, halogen, C₁-C₄-alkylcarbonyl, benzoyl, C₁-C₄-alkylcarbonylamino, benzoylamino, N-C₁-C₄-alkylphenylamino, C₁-C₄-alkoxycarbonyl, C₁-C₄-alkylsulfonyl, phenylsulfonyl, aminosulfonyl, aminocarbonyl, C₁-C₄-phenylalkyl and nitrobenzyl or R¹, R² or R², R³ or R³, R⁴ are a butadienediyl chain which is unsubstituted or substituted by R¹ to R⁴,
which comprises
a) reacting thiophenols of the general formula II 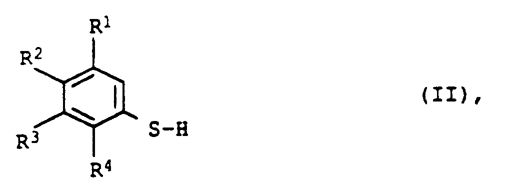 where the substituents R¹ to R⁴ have the abovementioned meanings, with chloroacetaldehyde at from 0 to 150°C, and
b) cyclizing the resulting (arylthio)acetaldehyde of the general formula III 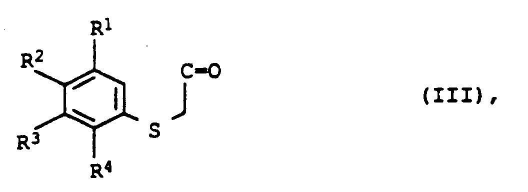 where the substituents R¹ to R⁴ have the above-mentioned meanings, by introducing into polyphosphoric acid or a mixture of phosphoric acid and phosphorus pentoxide at from 100 to 300°C and under from 0.001 to 1 bar.

2. A process for the preparation of benzo[b]thiophenes of the general formula I as claimed in claim 1, wherein water is removed from the reaction mixture by azeotropic distillation.

3. A process for the preparation of benzo[b]thiophenes of the general formula I as claimed in claim 1, wherein polyphosphoric acid is used for the cyclization.

4. A process for the preparation of benzo[b]thiophenes of the general formula I as claimed in claim 1, wherein a mixture of phosphoric acid and phosphorus pentoxide is used for the cyclization.

## Revendications

1. Procédé de préparation de benzo[b]thiophènes de la formule générale I : 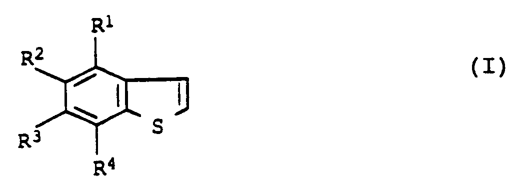 dans laquelle
les symboles R¹, R², R³ et R⁴ représentent, chacun indépendamment des autres, un atome d'hydrogène, un radical alkyle en C₁-C₄, alcoxy en C₁-C₄, halogénoalkyle en C₁-C₄, nitro, cyano, un atome d'halogène, un radical alkyl(C₁-C₄)carbonyle, benzoyle, alkyl(C₁-C₄)carbonylamino, benzoylamino, N-alkyl(C₁-C₄)phénylamino, alcoxy(C₁-C₄)carbonyle, alkylsulfonyle en C₁-C₄, phénylsulfonyle, aminosulfonyle, aminocarbonyle, phénylalkyle en C₁-C₄ ou nitrobenzyle, ou bien R¹, R², ou R², R³, ou R³, R⁴ représentent une chaîne butadiènediyle éventuellement substituée par des substituants R¹ à R⁴,
caractérisé en ce que
a) on fait réagir des thiophénols de la formule générale II : 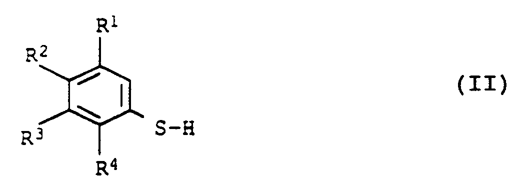 dans laquelle les substituants R¹ à R⁴ possèdent les significations qui leur ont été précédemment attribuées, avec le chloracétaldéhyde, à des températures de 0 à 150°C, et
b) on cyclise les (arylthio)acétaldéhydes formés de la formule générale III : 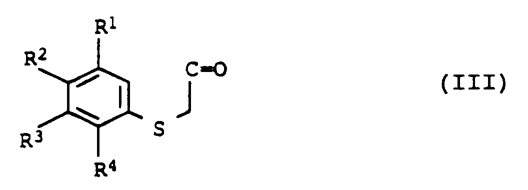 dans laquelle les substituants R¹ à R⁴ ont les significations qui leur ont été précédemment attribuées par introduction dans de l'acide polyphosphorique, ou dans un mélange constitué d'acide phosphorique et de pentoxyde de phosphore, à des températures de 100 à 300°C et sous des pressions de 0,001 à 1 bar.

2. Procédé de préparation de benzo[b]thiophènes de la formule générale I, selon la revendication 1, caractérisé en ce que l'on élimine l'eau du mélange réactionnel par distillation azéotropique.

3. Procédé de préparation de benzo[b]thiophènes de la formule générale I, selon la revendication 1, caractérisé en ce que l'on utilise de l'acide polyphosphorique pour procéder à la cyclisation.

4. Procédé de préparation de benzo[b]thiophènes de la formule générale I, selon la revendication 1, caractérisé en ce que l'on utilise un mélange d'acide phosphorique et de pentoxyde de phosphore pour procéder à la cyclisation.
